(19) Europäisches Patentamt / European Patent Office / Office européen des brevets

(11) EP 2 921 553 B1

## (12) EUROPEAN PATENT SPECIFICATION

(45) Date of publication and mention
of the grant of the patent:
**27.06.2018   Bulletin 2018/26**

(21) Application number: **14160765.5**

(22) Date of filing: **19.03.2014**

(51) Int Cl.:
*C12N 11/04* [(2006.01)]      *C12N 11/08* [(2006.01)]

---

(54) **Enzymatic reactor system**

Enzymatisches Reaktorsystem

Système de réacteur enzymatique

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.09.2015   Bulletin 2015/39**

(73) Proprietor: **Leibniz-Institut für Polymerforschung
Dresden e.V.
01069 Dresden (DE)**

(72) Inventors:
• **Ionov, Leonid**
  **01187 Dresden (DE)**
• **Dubey, Nidhi**
  **01069 Dresden (DE)**
• **Tripathi, Bijay**
  **01069 Dresden (DE)**
• **Stamm, Manfred**
  **01705 Pesterwitz (DE)**

(74) Representative: **Andrae | Westendorp
Patentanwälte Partnerschaft
Uhlandstraße 2
80336 München (DE)**

(56) References cited:
**GB-A- 2 215 335**

• **MD. TAIFUR RAHMAN ET AL: "Monodisperse Polymeric Ionic Liquid Microgel Beads with Multiple Chemically Switchable Functionalities",** LANGMUIR, vol. 29, no. 30, 30 July 2013 (2013-07-30) , pages 9535-9543, XP055128393, ISSN: 0743-7463, DOI: 10.1021/la401613w
• **NICOLE WELSCH ET AL: "Enhanced Activity of Enzymes Immobilized in Thermoresponsive Core-Shell Microgels",** THE JOURNAL OF PHYSICAL CHEMISTRY B, vol. 113, no. 49, 10 December 2009 (2009-12-10), pages 16039-16045, XP055128442, ISSN: 1520-6106, DOI: 10.1021/jp907508w
• **MAN FAI LEUNG ET AL: "New Route to Smart Core-Shell Polymeric Microgels: Synthesis and Properties",** MACROMOLECULAR RAPID COMMUNICATIONS, vol. 25, no. 21, 3 November 2004 (2004-11-03), pages 1819-1823, XP055046063, ISSN: 1022-1336, DOI: 10.1002/marc.200400362

---

EP 2 921 553 B1

## Description

### Technical field

The present invention relates to an enzymatic reactor system comprising an enzyme capable of catalyzing the synthesis of Acetyl-CoA immobilized on a polymer microgel, the use of the enzymatic reactor system for the synthesis of Acetyl-CoA and a method for the preparation of an enzymatic reactor system.

### Background of the Invention

In the era of nanotechnology, different materials are being explored for biological application. Likewise nano and submicron polymer particles have gained great interest in the field of biocatalysis. Latex particles are easy to synthesize, and their properties can be tuned as per the process requirement.

Poly N-isopropylacrylamide (PNIPAm) microgel particles are extensively studied thermo-responsive material due to pronounced thermal response near physiological temperature and well standardized synthetic protocols. Polyethylenimine is a cationic pH sensitive polymer and has been used as a stabilizing agent for enzymes. It provides multiple attachment point for enzyme and thus protects its quaternary structure. Enzymes such as trypsin, horse radish peroxidase, and others have previously been immobilized on the smart polymer (Kondo A. and Fukuda H., Journal of fermentation and bioengineering. 1997, 84, 337-341; Jing Xu, Fang Zeng, Shuizhu Wu, Xinxing Liu, Chao Hou and Zhen Tong, Nanotechnology. 2007, 18, 265704) etc.

S Acetyl CoA is central to biochemical reactions, metabolized for energy production in tri-citric acid cycle, providing precursor molecule in fatty acid synthesis, serving as starter units in polyketide synthesis etc. From a practical point of view, Acetyl CoA is a highly important biomolecule for biomedical studies such as drug discovery for metabolic disorders and biotechnological applications e.g. synthesis of lipids and polyketide-based anticancer drugs.

The high cost of Acetyl CoA makes these processes expensive and therefore different *in vitro* chemical and enzyme-based Acetyl CoA regeneration systems are employed within the main process. One such system is the use of enzyme Acetyl CoA synthetase (Acs; acetate: CoA ligase, EC 6.2.1.1), which catalyses the synthesis of acetyl CoA from acetate.

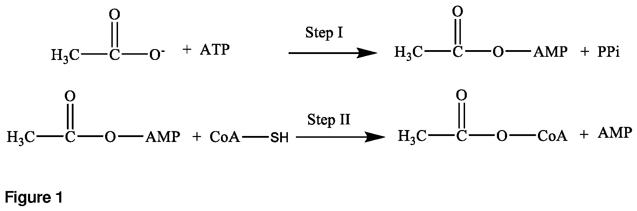

The main advantage of this enzyme is its high substrate specificity and the fact that the two step reaction is carried out by a single enzyme system as shown in Figure 1.

Despite all the advantages, the implementation of enzymes is not always straightforward, because a lot of issues arise during implementation. One of the major problems with the industrial application of enzymes is their lack of stability not only in temperature and pH extremes, but also under mechanical stress and in the presence of salts, alkalis and surfactants.

Furthermore, enzyme re-use is almost impossible and product quality is often adversely affected by enzyme inactivation steps and possibly also by difficult product purification schemes. Use of immobilized enzymes is supposed to improve the process economics by enabling enzyme re-use and enhancing overall productivity and robustness.

Among various types of supports for enzyme immobilization, membranes are considered to be good supports. Also, the immobilized enzyme-based bio-catalytic membrane reactors enable the integration of bio-catalysis and separation. Acetyl CoA synthetase immobilization on glass beads has previously been used for synthesis of $C^{11}$ labelled Acetyl CoA. The immobilization of enzyme on glass beads was screened for different cross linking methods and spacer arms.

According to an approach used in the prior art, CNBr activated glass beads gave maximum enzyme load and were selected for further optimization of the activity of the enzyme as well as column fabrication to obtain a complete conversion of 1 $\mu$mol of acetate (Mannens, G., Siegers, G., Lambrecht, R., Claeys, A. Biochimica et Biophysics Acta 1988, 959, 214-219; Mannens G., Slegers G., Lambrecht R., Goethals P., Radiopharmaceuticals 1988, Volume 25, Issue 7, pages 695-705.).

In other reports, Acetyl CoA synthetase was immobilized with a cascade of enzymes on Nafion membrane for studies of the citric acid cycle on a carbon electrode (Sokic-Lazic, D., Minteer, S. D. Biosensors and Bioelectronics. 2008, 24, 939-944) and patents have been filed wherein different physiologically active compounds including Acetyl CoA synthetase have been immobilized on the cellulose fibre Sepharose 4B (US 4610962 A, EP 0084975 A2, US 4960696 A).

GB 2215335 A discloses an immobilized enzyme conjugate soluble or dispersible in an organic solvent-containing medium, comprising a polymer microgel which has been prepared from at least one monomer, the or one of the monomers having been selected so as to introduce hydrophobic groups into the microgel, the microgel being conjugated via covalent linkages with the enzyme.

Man Fai Leung et al.: "New Route to Smart Core-Shell Polymeric Microgels: Synthesis and Properties", MACROMOLECULAR RAPID COMMUNICATIONS, vol. 25, no. 21, 3 November 2004 (2004-11-03), pages 1819-1823, discloses a new method for preparing smart polymeric microgels that consists of well-defined temperature-sensitive cores with pH-sensitive shells. Nicole Welsch et al.: "Enhanced Activity of Enzymes Immobilized in Thermoresponsive Core-Shell Microgels", THE JOURNAL OF PHYSICAL CHEMISTRY B, vol. 113, no. 49, 10 December 2009 (2009-12-12), pages 16039-16045, discloses a quantitative study of the catalytic activity of β-D-glucosidase from almons adsorbed on thermosensitive microgels. The core-shell particles used as a carrier system consist of a solid polystyrene core onto which a poly(N-isopropylacrylamide)(PNiPA) network ist grafted.

However, none of the studies of the prior art is able to provide an enzymatic reactor system based on responsive polymer microgel for the synthesis of Acetyl CoA which has a high enzymatic activity over a wide range of different temperatures and pH levels as well as for extended time spans, may be immobilized on membranes and can be re-used without a strong decline of enzymatic activity.

Accordingly, it is an object of the present intervention to provide an enzymatic reactor system which solves the above-mentioned problems. Furthermore, it is another object of the present invention to provide the use of such an enzymatic reactor system for the synthesis of Acetyl CoA and a method for the preparation of such a system.

## Summary of the Invention

These objects are solved by the aspects of the present invention as specified hereinafter.

According to the first aspect of the present invention, an enzymatic reactor system is providedcomprising an enzyme capable of catalyzing the reaction

$$A\ TP + Acetate + CoA \rightarrow AMP + Pyrophosphate + Acetyl\text{-}CoA \qquad (1)$$

immobilized on a polymer microgel, wherein preferably said enzyme is an Acetyl-coenzyme A synthetase, wherein the polymer microgel comprises a cationic polymer.

In one embodiment of the enzymatic reactor system, the enzyme capable of catalyzing reaction (1) is an enzyme having the EC number 6.2.1.1.

In another embodiment of the enzymatic reactor system, the enzyme capable of catalyzing reaction (1) is a polypeptide having at least 80% identity, preferably at least 90% identity, more preferably at least 95%, even more preferably at least 99% identity, to a polypeptide having an amino acid sequence as set out in SEQ ID NO: 1 or in SEQ ID NO: 2.

In another embodiment of the enzymatic reactor system, the enzyme capable of catalyzing reaction (1) has the amino acid sequence as set out in SEQ ID NO: 1 (Acetyl-coenzyme A Synthetase 1; ACS1 from *Saccharomyces cerevisiae*) or in SEQ ID NO: 2 (Acetyl-coenzyme A Synthetase 2; ACS2).

In one embodiment of the enzymatic reactor system, the enzyme capable of catalyzing reaction (1) immobilized on a polymer microgel is supported on a membrane, preferably on a polyethylene terephthalate (PET) track etched membrane.

According to the invention, the polymer microgel comprises a cationic polymer.

In yet another embodiment of the enzymatic reactor system, the polymer microgel comprises aminoethylmethacrylate and/or polyethyleneimine.

In one embodiment of the enzymatic reactor system, the polymer microgel comprises at least 0.001 wt%, preferably at least 0.05 wt%, more preferably at least 0.1 wt% of aminoethylmethacrylate and/or polyethyleneimine.

In another embodiment of the enzymatic reactor system, the polymer microgel comprises at most 5 wt%, preferably at most 1 wt%, more preferably at most 0.5 wt% of aminoethylmethacrylate and/or polyethyleneimine.

In another embodiment of the enzymatic reactor system, the polymer microgel further comprises poly(N-isopropylacrylamide).

In one embodiment of the enzymatic reactor system, the enzyme capable of catalyzing reaction (1) is immobilized on the polymer microgel by covalent linkage or ionic adsorption.

In one particular embodiment of the enzymatic reactor system, the enzyme capable of catalyzing reaction (1) is covalently linked to the microgel.

In a particular embodiment of the enzymatic reactor system, the enzyme capable of catalyzing reaction (1) is covalently linked to functional groups on the surface of the microgel, preferably the enzyme is covalently linked to amino groups on the surface of the microgel, more preferably the enzyme is covalently linked to amino groups on the surface of a microgel comprising aminoethylmethacrylate.

In an alternative embodiment of the enzymatic reactor system, the enzyme capable of catalyzing reaction (1) is immobilized on the microgel by ionic adsorption.

In one embodiment of the enzymatic reactor system, the polymer microgel is of the core-shell type.

According to the second aspect of the present invention, the use of the enzymatic reactor system is provided for the synthesis of Acetyl-CoA.

According to one embodiment of the second aspect of the present invention, the system is used in a dead end filtration device.

According to the third aspect of the present invention, a method for the preparation of an enzymatic reactor system according to the first aspect of the present invention is provided, wherein the immobilization of the enzyme capable of catalyzing reaction (1) on a polymer microgel is performed by covalent linkage or ionic adsorption.

## Description of Figures

Figure 1 shows the two step synthesis reaction of acetyl CoA from acetate catalyzed by acetyl CoA synthetase.

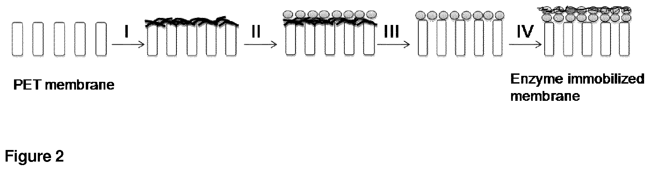

Figure 2 shows the fabrication of a membrane covered with immobilized enzyme microgel. Step I: Deposition of $Cd(OH)_2$ nanostrand on PET track etched membrane, II: Microgel layer deposition on nanostrand, III: Removal of nanostrand via acid dissolution and cross-linking with glutaraldehyde, IV: Immobilization of bioconjugate on microgel layer.

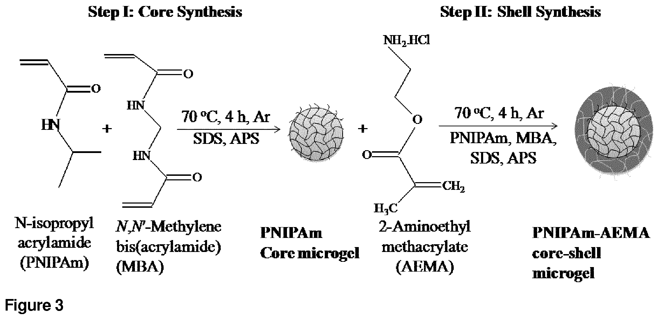

Figure 3 shows a schematic representation of the synthesis of a PNIPAm-AEMA core shell microgel.

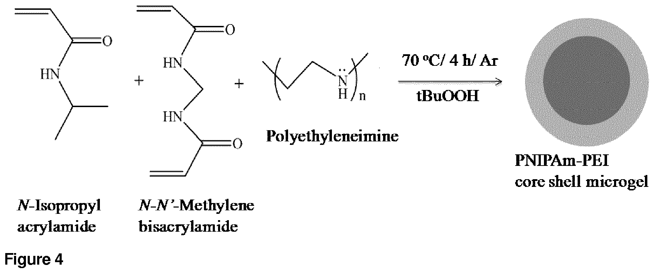

Figure 4 shows a schematic representation of the preparation of a PEI-PNIPAm microgel.

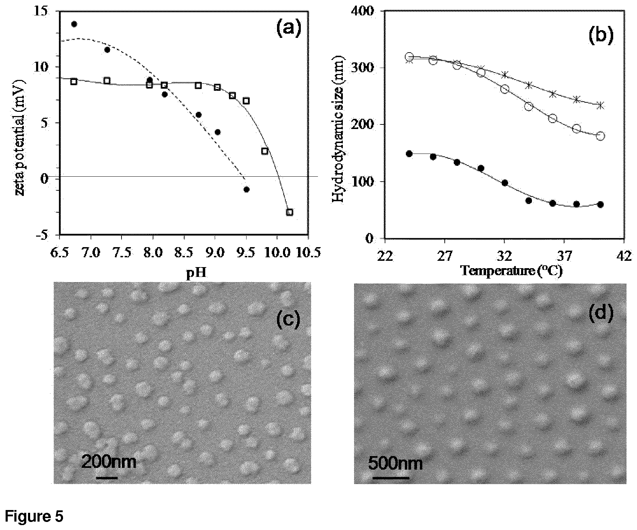

Figure 5 shows the characterization of core-shell microgel particles: (a) Zeta potential of PNIPAm-AEMA (squares) and PNIPAm-PEI(circles) microgels as a function of pH. (b) Change in hydrodynamic radius with respect to temperature of PNIPAm core (black circles), PNIPAm-AEMA core shell microgel (white circles) and PNIPAm-PEI microgel (cross marks) as determined by DLS; Scanning electron microscopy images of (c) PNIPAm-AEMA and (d) PNIPAm-PEI core shell microgel after vacuum drying at room temperature.

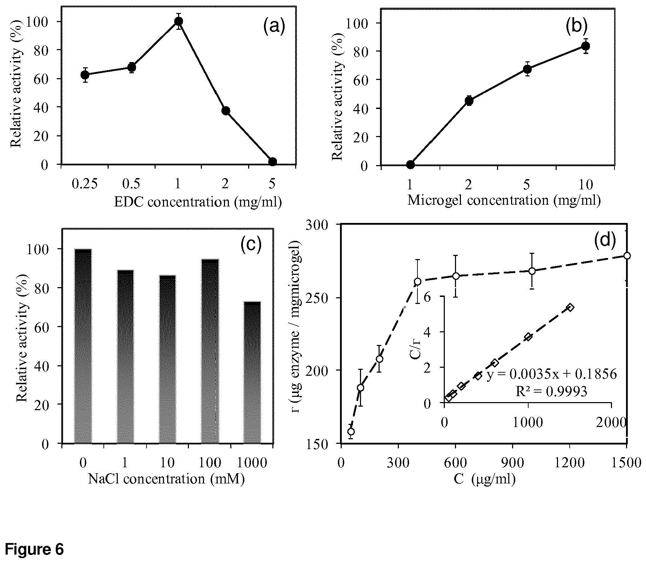

Figure 6 shows the standardization of immobilization parameters by checking (a) effect of EDC, (b) microgel and (c) salt concentrations on activity of the enzyme (in case of PNIPAM-AEMA). (d) Adsorption isotherm of acetyl-CoA synthetase on PEI-PNIPAm microgel, the adsorbed amount of enzyme per mg of PEI-PNIPAm microgel ($\Gamma$) is plotted versus the concentration of free enzyme in solution (C); the dashed line represents the fit of the experimental data by the Langmuir isotherm; the inset displays the data as a linearized Langmuir plot according to equation (6). Relative activities are normalized activities of enzymes with respect to highest activity of respective enzymes and experiment. Each experiment was done in duplicate, error bars in figures show standard deviations.

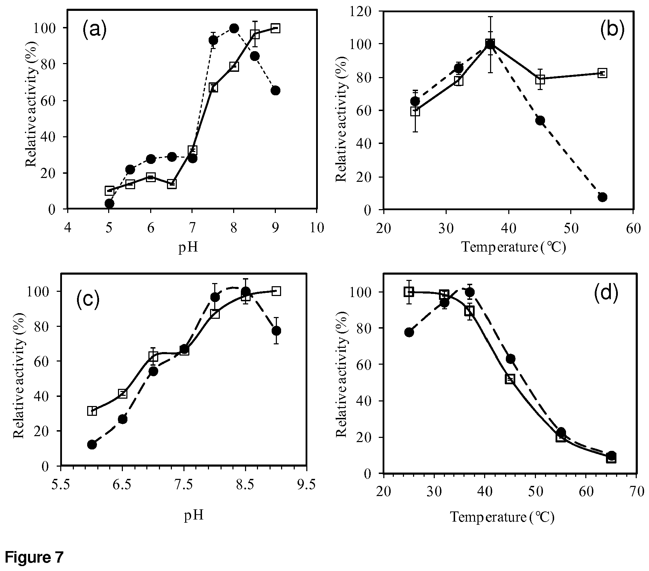

Figure 7 shows the effect of pH (a) and temperature (b) on the activity of free (circles) and immobilized acetyl CoA synthetase on PNIPAm AEMA microgels (squares). (c) shows the effect of pH and (d) temperature on the activity of free (circles) and immobilized acetyl CoA synthetase on PNIPAm AEMA microgels (squares).

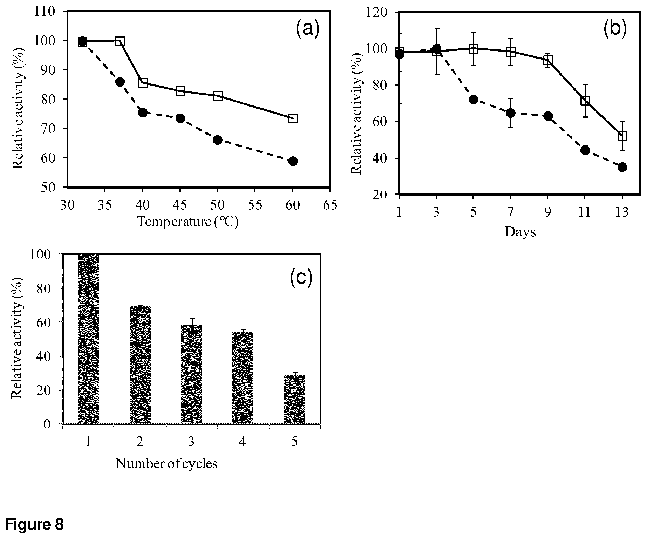

Figure 8 shows the stability profile of free (circles) and PNIPAm-AEMA-immobilized acetyl CoA synthetase (squares). (a) Thermal stability was determined by measuring the activity after incubation at respective temperatures for 10min and subsequently cooling on ice. (b) Storage stability at 4°C was monitored by checking the activity of free and immobilized acetyl CoA synthetase in borate buffer (0.1 M, pHS). (c) Operation stability of PNIPAm-AEMA-immobilized acetyl CoA synthetase was checked for 5 cycles at standard reaction conditions. Relative activities are normalized activities of enzymes with respect to the highest activity of respective enzymes and experiment. Each experiment was done in triplicate and error bars in figures show standard deviations calculated with three data points.

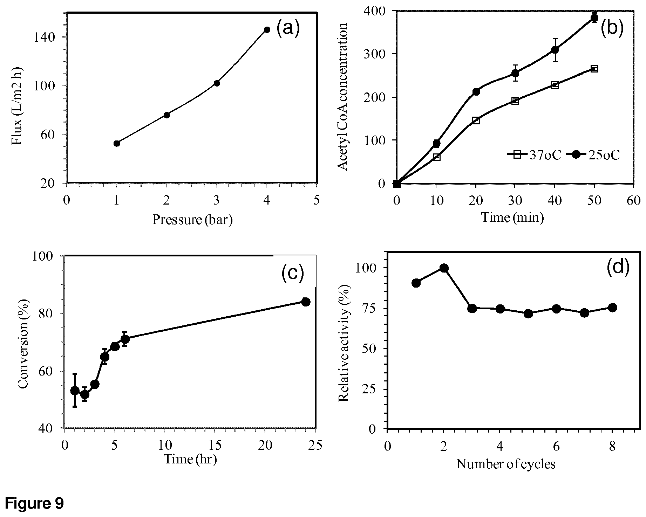

Figure 9 shows the performance of enzyme bioreactor (a): Flux versus pressure relation of the bioconjugate-PET membrane; (b): Effect of temperature on the membrane performance over time at 25°C (squares) and 37°C (circles). (c) Bioconversion study of 1.5 U bioconjugate-PET membrane over a period of 24 hr at 25°C. (d) Operational stability of membrane at 25°C.

## Detailed Description of the Invention

It has been surprisingly found that the immobilization of an enzyme capable of catalysing the reaction

$$ATP + Acetate + CoA \rightarrow AMP + Pyrophosphate + Acetyl\text{-}CoA \qquad (1)$$

on a polymer microgel according to claim 1 highly stabilizes the enzyme, leading to increased activity over time, at a wide range of temperature and pH and even allows the effective re-use of the enzymatic reactor system over multiple cycles without significant loss of enzyme activity.

The present invention relates to an enzymatic reactor system comprising an enzyme capable of catalyzing the above reaction (1) for the synthesis of Acetyl CoA. Herein, the term "an enzyme capable of catalyzing the reaction (1)" means that the enzyme functions as a catalyst in said reaction (1). A catalyst, as is generally appreciated in the art, is a substance that increases the rate of a chemical reaction without itself undergoing any permanent chemical change. Accordingly, a substance which increases the reaction rate of reaction (1) without undergoing any permanent chemical change may be considered to be an enzyme capable of catalyzing the reaction (1).

According to a preferred embodiment, the enzyme is an Acetyl-coenzyme A synthetase. Acetyl-coenzyme A synthetases are generally classified under EC number 6.2.1.1. of the Enzyme Number Commission (EC) classification scheme. Thus, according to one embodiment of the present invention, the enzyme is an enzyme having the EC number 6.2.1.1.

The model organism *Saccharomyces cerevisiae* or baker's yeast is widely used for research purposes and large-scale production of enzymes, such as insulin or others. *S. cerevisiae* contains two different structural genes each encoding an active Acetyl-coenzyme A synthetase, ACS 1 and ACS 2. The amino acid sequence of ACS 1 is referred to herein as SEQ ID NO: 1 and the amino acid sequence of ACS 2 is referred to herein as SEQ ID NO: 2.

According to one embodiment of the present invention, the enzyme capable of catalyzing reaction (1) is a polypeptide having at least 80% identity to a polypeptide having an amino acid sequence as set out in SEQ ID NO: 1, preferably at least 90% identity, more preferably at least 95% identity, even more preferably at least 99% identity.

According to another embodiment, the enzyme capable of catalyzing reaction (1) is a polypeptide having at least 80% identity to a polypeptide having an amino acid sequence as set out in SEQ ID NO: 2, preferably at least 90% identity, more preferably at least 95% identity, even more preferably at least 99% identity.

For the purpose of this invention, it is defined here that in order to determine the percent identity of two amino acid sequences, the sequences are aligned for optimal comparison purposes. In order to optimize the alignment between the two sequences gaps may be introduced in any of the two sequences that are compared. Such alignment can be carried out over the full length of the sequences being compared.

A comparison of sequences and determination of percent identity between two sequences can be accomplished using a mathematical algorithm. The skilled person will be aware of the fact that several different computer programs are available to align two sequences and determine the homology between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The term "% identity" is defined as the percentage of amino acids in a candidate sequence that are identical with the amino acids in a reference nucleic acid sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

For purposes herein, the % sequence identity of a given amino acid sequence C with a given nucleic acid sequence D (which can alternatively be phrased as a given sequence C that has or a certain % sequence identity with a given sequence D) is calculated as follows:

$$100 \times W/Z \qquad (2)$$

where W is the number of amino acids scored as identical matches by the sequence alignment program in that program's alignment of C and D, and where Z is the total number of amino acids in D. It will be appreciated that where the length of sequence C is not equal to the length of sequence D, the % sequence identity of C to D will not equal the % sequence identity of D to C.

According to a preferred embodiment, the enzyme capable of catalyzing reaction (1) has the amino acid sequence as set out in SEQ ID NO: 1, corresponding to the amino acid sequence of Acetyl-coenzyme A synthetase 1 from *Saccharomyces cerevisiae.* In particular, the S-Acetyl-coenzyme A synthetase from baker's yeast (*S. cerevisiae*) as distributed by Sigma-Aldrich, St. Louis, USA may be used.

According to another preferred embodiment, the enzyme capable of catalyzing reaction (1) has the amino acid sequence as set out in SEQ ID NO: 2, corresponding to the amino acid sequence of Acetyl-coenzyme A synthetase 2 from *Saccharomyces cerevisiae.*

According to the invention, the polymer microgel comprises a cationic polymer. In particular, the polymer microgel comprises aminoethylmethacrylate (AEMA) and/or polyethyleneimine. A polymer microgel comprising aminoethylmethacrylate (AEMA) and/or polyethyleneimine herein means a polymer microgel comprising structural units based on the polymerization of AEMA monomers and/or polyethyleneimine units. According to a preferred embodiment, the polymer microgel comprises a cationic copolymer which is obtained by copolymerization of AEMA with additional monomers.

In one embodiment of the enzymatic reactor system, the polymer microgel comprises at least 0.001 wt%, preferably at least 0,05 wt%, more preferably at least 0,1 wt% of aminoethylmethacrylate and/or polyethyleneimine.

In another embodiment of the enzymatic reactor system, the polymer microgel comprises at most 5 wt%, preferably at most 1 wt%, more preferably at most 0.5 wt% of aminoethylmethacrylate and/or polyethyleneimine.

According to another embodiment, the polymer microgel further comprises poly(N-isopropylacrylamide).

According to the present invention, the enzyme capable of catalyzing reaction (1) may be immobilized on the polymer microgel by covalent linkage or by ionic adsorption. According to one embodiment, the enzyme is covalently linked to the microgel. Preferably, the enzyme is covalently linked to functional groups on the surface of the microgel, more preferably the enzyme is covalently linked to amino groups on the surface of the microgel, even more preferably the enzyme is covalently linked to amino groups on the surface of a microgel comprising aminoethylmethacrylate.

It is known from the active site structure of acetyl CoA synthetase that a lysine residue is very critical in the first step of reaction where acetate is activated to acetyl phosphate. Therefore, for covalent conjugation of enzyme to microgel, carboxylic acid residues of the enzyme were targeted using the zero length crosslinker EDC. Thus, the enzyme may be immobilized on the polymer microgel by covalent linkage using carbodiimide chemistry, in particular using 1-ethyl-3-(3-NNdimethylaminopropyl) carbodiimide (EDC) chemistry. In this way, carboxyl groups of the enzyme are linked to the primary amines present in the aminoethylmethacrylate moieties.

According to an alternative embodiment, the enzyme capable of catalyzing reaction (1) is immobilized on the polymer microgel by ionic adsorption. According to a preferred embodiment, the enzyme is immobilized by ionic adsorption to a microgel comprising a cationic polymer, preferably polyethyleneimine. According to an alternative embodiment, the enzyme is adsorbed to a microgel comprising aminoethylmethacrylate.

According to one embodiment, the enzyme capable of catalyzing reaction (1) immobilized on a polymer microgel is supported on a membrane. Such an enzyme membrane reactor serves as a good platform for biocatalysis and bioseparation. In an enzymatic membrane reactor, the membrane governs the mass transport across itself, thus also retaining the enzymes inside the reactor and achieving a certain level of product separation as well. Further, the continuous removal of product can shift the equilibrium of a reaction towards the product side and thereby increase the productivity of the whole process, which is a remarkable advantage of such a membrane reactor.

A preferred membrane to be used in the present invention is a PET track etched membrane. As a PET track etched membrane, PET track-etched membranes with a pore size of 300 - 400 nm and diameter of 25 mm as available from Whatman Co., GE Healthcare Europe, Freiburg, Germany may be used.

The enzyme immobilized membrane surface was prepared on a polyethylene terephthalate (PET) track etched membrane support using an enzyme immobilized microgel. The membrane fabrication strategy is outlined in Figure 2.

The pores of the PET membrane were first covered with $Cd(OH)_2$ nanostrands, which was prepared by mixing CdCl2 and an aminoethanol solution (see for example Qiugen Zhang et al., Ultrathin freestanding nanoporous membranes prepared from polystyrene nanoparticles, J. Mater. Chern., 2011, 21, 1684).

The nanostrand solution (10 mL) was suction filtered on the PET membrane. Subsequently, a thin layer of microgel was prepared by filtering a 1 mg/ml concentration microgel solution and crosslinking by glutaraldehyde. After microgel crosslinking, the sacrificial cadmium hydroxide nanostrand layer was removed by repeated passing of 10 mM HCl solution.

The membrane was extensively rinsed with buffer and finally 100 $\mu$l of (1.5 U) enzyme immobilized microgels were diluted in buffer and allowed to crosslink with free glutaraldehyde at 4°C for overnight on the prepared surface. The membrane was washed to remove the unbound bioconjugate. Such fabricated membrane may be preferably used in a dead end filtration device, for example to check the catalytic performance.

It is assumed that the enzyme capable of catalyzing the reaction (1) is stabilized by the immobilization on the polymer microgel which possibly influences the reaction characteristics. Further, it is thought that a net positive charge of the polymer microgel may positively influence the activity of enzymes which carry a net negative charge, as is the case for the enzymes encoded by SEQ ID NO: 1 and 2. Thus, the immobilization by covalent linkage or ionic adsorption to microgels comprising a cationic polymer is particularly preferred for enzymes which carry an overall negative charge. Immobilization of the enzyme by ionic adsorption is particularly advantageous since the enzyme is not permanently chemically changed, e.g. by crosslinking of the enzyme's carboxyl groups.

According to one embodiment of the present invention, the polymer microgel is of the core-shell type. Core-shell type microgels have the advantage of carrying a high density of functional groups on the surface and hence a high load of molecules can be immobilized.

The core-shell microgel particles may be synthesized by a two-step free radical precipitation polymerization method or by graft polymerization of the cationic polymer.

The two-step free radical precipitation polymerization method may be performed as shown in Figure 3. This method is preferably used for the synthesis of a polymer microgel comprising aminoethylmethacrylate as the cationic component.

A monomer solution of NIPAm and MBA may be taken for the synthesis of the core of the particle, preferably in a ratio of 98:2. For shell synthesis, a solution of NIPAm, MBA, and AEMA may be used, preferably in the ratio of 96.5:2:1.5.

Alternatively, the core-shell microgel particles may be synthesized by graft polymerization of the cationic polymer as shown in Figure 4. The microgel may be prepared by the graft copolymerization of NIPAM from PEI in presence of tert-butyl hydroperoxide (tBuOH) as an initiator. tBuOH reacts with amine groups of PEI chains and generates amino and tBuO free radicals. These free radicals initiate the graft copolymerization and the homopolymerization of NIPAM in the presence of a cross-linker, N,N-methylenebisacrylamide.

At the reaction temperature, the PNIPAm chain becomes hydrophobic while PEI chains remain hydrophilic. These amphiphilic PEI-g-PNIPAM formed promote further emulsion polymerization of NIPAm by self-assembly to form core-shell microgel particles.

**Examples**

The following materials have been used for the Examples as described below:
N-isopropylacrylamide (97 %) (NIPAm) was recrystallized from n-hexane and dried in vacuum before use. Branched poly(ethyleneimine) (PEI) with an average molecular weight of 25,000 (50 wt.% solution in water), N,N- methylene bisacrylamide (≥99.5%) (MBA), sodium dodecyl sulfate (SDS), ammonium persulfate (~98.0%) (APS), 2-Aminoethyl methacrylate hydrochloride (90 %) (AEMA), 1-ethyl-3-(3-NN-dimethylaminopropyl) carbodiimide (EDC), *tert-butyl* hydroperoxide (70% solution in water, tBuOOH), S-Acetyl-coenzyme A synthetase (EC 6.2.1.1, >3units/mg, from baker's yeast) (ACS), Malic acid Dehydrogenase from porcine heart (≥600 units/mg protein), Citrate Synthase from porcine heart (≥100 units/mg protein), and all other chemicals were obtained from Sigma-Aldrich (Germany).

BCA Protein Assay Kit (Thermo Scientific (Pierce protein)), Acetyl-Coenzyme A (Roche, Germany), β-Nicotinamide adenine dinucleotide and NADH disodium salt (Carl Roth, Germany), were purchased from respective companies. 30 and 10 kDa molecular weight cut off (MWCO) centrifugal filter tubes were purchased from Millipore. All the chemicals were used as received or else stated. Water used throughout the investigation was purified to a resistance of 18 MΩ (Millipore), and filtered through a 0.45 μm nylon filter to remove particulate matter.

Example 1

Synthesis of PNIPAM-AEMA

The core shell microgel particles were synthesized by two step free radical precipitation polymerization method as shown in Figure 3. A 64 mM total monomer concentration of NIPAm and MBA in 98:2 ratio was used for the synthesis of the core. For shell synthesis, a 37 mM solution of NIPAm, MBA, and AEMA in the ratio of 96.5:2:1.5 was used. Except for the initiator all ingredients were first dissolved in water and filtered through Whatman filter paper and heated under stirring to 70°C under a gentle stream of Argon for 1 h.

First, the core was synthesized by rapid addition of 1 ml of APS solution (2 mM) into the monomer solution containing SDS (2 mM). The reaction was allowed to proceed for 4 h at 70°C under continuous stirring. After polymerization, the solution was filtered through 0.45 μm pore size filter. Next the shell synthesis was done by addition of surfactant SDS (0.7 mM) to 20 mL core solution and heated under stirring in nitrogen gas atmosphere to 70 C.

Further, 25 mL of monomer solution was added to the heated core microgel solution and volume was adjusted to 100 mL. The reaction was initiated with the addition of 1 ml of an APS solution (1.5 mM) and reaction mixture was continuously stirred for 4 h at 70°C. The obtained core-shell microgel solution was allowed to cool at room temperature and filtered with a suitable membrane. The unreacted monomers and small molecular weight polymers were removed from the microgel solution by continuous dialysis for a week using a 10 KDa MWCO dialysis tube against a daily change of water.

Example 2

Synthesis of PEI-PNIPAM

The cationic thermo-responsive PNIPAm- PEI microgel was synthesized via graft polymerization of PEI as shown in Figure 4. PEI (0.4 g, 50% solution) solution in water was initially neutralized using 1M of HCl solution. In a typical solution of 50 mL, a mixture of NIPAM monomer (800 mg) and MBA (80 mg) was separately treated under a gentle stream of argon for 30 min at 70°C and then charged to the PEI solution in a triple neck flask.

Dilute tert-butyl hydroperoxide solution (0.5 mL, 10 mM) was added dropwise to the mixture to initiate polymerization reaction, and the solution was stirred at 70°C for 2 h under Argon. After the reaction, the dispersion of microgels was carefully purified by repeated centrifugation at 13,000 rpm for 30 min and further purified by dialysing against water for one week at room temperature. Lyophilized microgel was dispersed to concentration of 1 wt%. The microgel suspensions were shaken for 24 h to receive an evenly dispersed microgel solution.

Example 3

Dynamic Light Scattering, Zeta potential measurements and Scanning electron microscopy of PNIPAM-AEMA and PNIPAm-PEI microgels

The Dynamic light scattering (DLS) measurements at different temperatures were performed for the microgels to analyse the particle size and its thermo-responsive behaviour. Zeta potential measurements of particles were also carried out to identify the charge behaviour with respect to pH. Both, DLS and zeta potential experiments were done using Zeta sizer Nano 3000HS (Malvern Instruments/UK), equipped with a 633 nm He/Ne laser and a non-invasive back scatter (NIBS®) technology. In the case of PNIPAM-AEMA microgels, measurements were performed for both core and core-shell microgels.

Before the size measurements, samples were thermally equilibrated for 10 min and data were acquired by averaging 30 measurements, with a 10 s integrating time for each measurement. Volume phase transition temperature was determined with respect to temperature (24 to 40°C). Zeta potential was obtained from pH range 6 to 11 and the values were average of three successive readings.

Furthermore, the morphology of synthesized microgel was also characterized by scanning electron microscopy. SEM was performed on vacuum dried microgels on silicon wafer using a NEON 40 FIB-SEM workstation (Carl Zeiss AG, Germany) operated at 3 kV, after 3 nm thick sputter coating of platinum.

The cationic nature of prepared core shell microgels was studied by measuring the zeta potential of microgel solution. The change in zeta potential of microgel as a function of pH is depicted in Figure 5a. The zeta potential was positive at a wide range of pH and the isoelectric point of PNIPAM-AEMA and PEI-PNIPAM microgels was found to be around pH 10. This characteristic charge of the PNIPAM-AEMA microgels is due to the presence of free -NH2 groups in the shell added by cationic monomer AEMA whereas PEI polymer contributes to the charge for the PEI-PNIPAM microgel structure. Thus, the positive value of zeta potential confirms that the PNIPAM-AEMA and PEI-PNIPAM microgels were sufficiently cationic.

The temperature dependent size behavior of microgels are depicted in Figure 5b. For PNIPAM-AEMA microgel size of core and core-shell microgels in water at 24°C was found to be 150 nm and 320 nm. At 40°C, however, the size changed to 60 and 180 nm for core and core-shell microgel particles, respectively. The size of PEI-PNIPAM microgels in water at 24°C was found to be 320 nm, while at 40°C, the size reduced to 234 nm.

PNIPAm exhibits a reversible transition from a solvated random coil to a desolvated globular state at 32°C, due to the disruption of hydrogen bonds and dominance of the hydrophobic part of PNIPAm at high temperature, causing water to expel out of its structure.

The PNIPAm microgels possess a volume phase transition from a swollen state to a collapsed state at or near this temperature. Furthermore, this transition temperature is also affected by the presence of functional comonomer in the structure.

Figure 5b confirms that the synthesized PNIPAM-AEMA and PEI-PNIPAM microgel particles exhibit a phase transition at around 32°C which is near to the LCST of PNIPAm. The morphological characteristics of dried PNIPAM-AEMA and PEI-PNIPAM microgels were imaged with the help of SEM and the image is shown in Figure 5c & d respectively. It is evident from the SEM image that the PNIPAM-AEMA microgels were of nearly uniform size and shape.

Example 4

Immobilization of Acetyl CoA-synthetase on PNIPAM-AEMA microgels

Acetyl CoA-synthetase was covalently immobilized to PNIPAm-AEMA core-shell microgel using carbodiimide chemistry in 0.1 M Borate buffer at pH 8. Inorganic phosphate present in commercial enzyme preparation (interferes with molybdate assay) was removed by filtration using 30 kDa centrifuge filter tubes (Millipore).

The immobilization conditions were standardized before conjugation with respect to EDC, PNIPAm-AEMA microgel, and salt concentration. The effect of EDC concentrations on Acetyl CoA-synthetase immobilization to microgel were studied from 0.25 to 5 mg/ml concentration. As shown in Figure 6 (a), the enzyme activity increased with increase in EDC concentration up to 1 mg/ml, above this concentration there was a sharp decline in enzyme activity. Since EDC is a crosslinker, at higher concentration, it crosslinks enzyme molecules to one another at an increasing rate leading to decrease in enzyme activity.

To determine the working concentration of PNIPAm-AEMA microgel for enzyme immobilization, different concentrations of PNIPAm-AEMA microgel from 1 to 10 mg/mL were incubated with 1 mg/ml of enzyme in presence of EDC. The highest concentration of microgel for this study was maintained to be 10 mg/mL to avoid a very viscous solution above this concentration. From Figure 6 (b), it can be seen that the enzyme activity increases with increase in PNIPAm-AEMA microgel concentration. Therefore for further use, a working concentration of 10 mg/mL PNIPAm-AEMA microgel was found suitable, which is a 1:10 proportion of enzyme to microgel for conjugation protocol.

To obtain the bioconjugate after the immobilization process, the PNIPAm-AEMA microgels were precipitated from the reaction mixture at room temperature with the help of salt. Hence, it was important to study the effect of salt concentration on enzyme activity. The conjugate was subjected to different concentration of NaCl solution and activity was determined.

The activity of free acetyl CoA synthetase and their bioconjugates with PNIPAm-AEMA or microgel was studied in borate buffer (0.1 M, pH 8), in presence of 20 mM potassium acetate, 4 mM $MgCl_2$, 4 mM glutathione, 1 mM ATP, and 500 $\mu$M coenzyme A. The reaction was terminated by addition of acidic molybdate reagent. The pyrophosphate-molybdate complex formed was further reduced by addition of mercaptoethanol and Eikonogen reagent. The obtained coloured product was quantified by measuring the absorption at 590 nm and pyrophosphate concentration was determined ($\varepsilon$ = 2.7 x $10^4$ $M^{-1}$ $cm^{-1}$).

One unit of enzyme activity was defined as the amount of enzyme required to form 1 $\mu$mol pyrophosphate per min at pH 8 and 37°C. Bradford micro assay with a sensitivity of 1-10 $\mu$g/mL was used to determine protein concentration using bovine serum albumin as the standard protein.

At a given centrifugal condition, 1 M NaCl solution was the optimum to obtain the complete bioconjugate. Also, Figure 6 (c) shows that no significant reduction in enzyme activity at 1 M NaCl concentration was observed as compared to 10-1000 folds less concentration of NaCl. A total 6 ml of mixture solution containing microgel solution (10 mg/ml), enzyme solution (1 mg/ml), and EDC (1 mg/ml) were incubated at 10°C for 4 h. After completion of the incubation period, an equal volume of tris-Cl buffer (0.1 M, pH 8) was added to the reaction mixture to quench extra EDC. The bioconjugate formed was precipitated by adding salt solution (1 M NaCl), and obtained by centrifugation at 12,000 rpm for 5 min at 25°C.

Finally the bioconjugate was desalinated by repeated centrifugation with borate buffer using 10 kDa MWCO centrifuge filter tubes. The amount of enzyme loaded to the PNIPAM-AEMA microgel was determined by subtracting the residual protein content in the supernatant from the initial protein content. The enzyme and bioconjugate were stored in 0.1 M borate buffer containing 1 mM glutathione and 1 mM magnesium chloride (pH 8).

The number of molecules per unit volume ($N_m$ and $N_e$) for microgel and enzyme were calculated using the following formula

$$N = \frac{V}{v_i} \qquad\qquad (3)$$

where $V$ is the total volume of particles per unit volume of dispersion (mL) and $v_i$ is the mean volume of a particle. The value of $V$ was found as

$$V = \frac{W}{\rho} \qquad\qquad (4)$$

$W$ is the mass of wet microgel or enzyme, $\rho$ is the density, since the microgel particles were highly swollen in water $\rho$ for microgel was assumed to be 1.00 g $mL^{-1}$ and average protein density for enzyme 1.35 g $mL^{-1}$. The value of $v_i$ was determined as

$$vi = \left(\frac{4}{3}\right) \pi \ R_h{}^3 \qquad\qquad (5)$$

where $R$h is the hydrodynamic radius. Using these data the number of enzyme molecules on single microgel particles was calculated from $N_e /N_m$.

After standardization of immobilization conditions, the conjugation was carried out at optimized conditions. From the Bradford method, the amount of Acetyl CoA-synthetase immobilized onto PNIPAm-AEMA microgel was determined to be 68% of the initial Acetyl CoA-synthetase, suggesting an amount of immobilized enzyme of 0.02 mg per mg of dry microgel particles. From this data ~24 Acs molecules were calculated to be bound on the surface of each microgel particle.

The activity of immobilized Acetyl CoA-synthetase was found to be 0.23 mU/mg of carrier which was about 61.55% of the initial activity of Acs. The attachment of the enzyme to the PNIPAm-AEMA microgel using carbodiimide chemistry resulted in an enzyme with a higher activity with respect to the enzyme bound in total. For CNBr activated glass beads, as published in the prior art, 100% enzyme immobilization was achieved but only 23% of enzyme was reported to be active (Mannens, G., Siegers, G., Lambrecht, R., Claeys, A. Biochimica et Biophysics Acta 1988, 959, 214-219).

The decrease in enzyme activity after immobilization is effect of several factors such as a change in enzyme conformation or modification of important residues due to covalent immobilization and addition to this immobilization matrix also imparts additional factors like steric hindrance, partition effects and mass transfer constraints.

Example 5

Immobilization of Acetyl CoA-synthetase on PEI-PNIPAM microgels

The immobilization of Acetyl CoA-synthetase on PEI-PNIPAm microgel was achieved via adsorption in 0.1 M Tris-Cl buffer at pH 8 for 6 h at 4°C. The conjugate was obtained by centrifugation at 12,000 rpm for 15 min at 25°C and subsequently washed with the same buffer to remove non adsorbed enzymes. Using Micro BCA (for protein concentrations lower than 20 $\mu$g/mL) assay kits, the amounts of adsorbed enzyme loaded to the microgel was determined by subtracting the residual protein content in the supernatant from the initial protein content. The enzyme and conjugate were stored in 0.1 M Borate buffer containing 1 mM glutathione and 1 mM magnesium chloride pH 8 for further use.

The activity of free and immobilized Acs was determined spectrophotometrically by following acetyl CoA synthesis at 340 nm wavelength by coupling to Citrate synthase (CS) and malate dehydrogenase (MDH) assay.

The assay medium composition for enzyme activity consists of 20 mM potassium acetate, 4 mM MgCl, 4 mM glutathione, 1 mM ATP, 500 $\mu$M co-enzyme A, 1 mM NAD and 4 mM malate in 100mM Tris Cl buffer pH 8. The acetyl CoA synthesized was correlated to the concentration of NADH formed in a coupled assay using $\epsilon_{340}$ = .6220 M$^{-1}$ cm$^{-1}$. One unit of enzyme activity was defined as the amount of enzyme which is required to form 1 $\mu$mol of NADH per min at pH 8 and 37°C.

Preliminary experiments were performed to determine optimal conditions like pH, temperature and microgel concentration for enzyme coupling. The studies were done at standard conditions of a microgel concentration of 10 mg/ml, a temperature <10°C and pH 8.0. The adsorption isotherm studies were carried out by investigating the Acetyl CoA-synthetase binding to the microgel as a function of enzyme concentrations.

Figure 6(d) suggests that the adsorption behavior of Acetyl CoA-synthetase on the PEI-PNIPAm microgel follows a Langmuir-type model which can be described by the following Langmuir isotherm equation (6).

$$\Gamma = \frac{\Gamma_{max}C}{K_d + C} \qquad \Gamma = \frac{\Gamma_{max}c}{K_d + c} \qquad\qquad (6)$$

The Langmuir isotherm equation can be linearized by multiplying both sides by $(K_d + C)$ and dividing by $\Gamma$, resulting in equation 5 with which the experimental data are fit as shown in (Figure 6(d) insert).

$$\frac{C}{\Gamma} = \left(\frac{1}{\Gamma_{max}}\right)\left(C + \frac{K_d}{\Gamma_{max}}\right) \qquad \frac{c}{\Gamma} = \left(\frac{1}{\Gamma_{max}}\right)\left(c + \frac{K_d}{\Gamma_{max}}\right) \qquad (7)$$

where $\Gamma$ is the amount of Acetyl CoA-synthetase adsorbed on the PEI-PNIPAm microgel ($\mu$g/mg), $\Gamma_{max}$ is the maximum binding capacity ($\mu$g/mg), C is the Acetyl CoA-synthetase concentration in solution ($\mu$g/mL), and $K_d$ is the dissociation constant ($\mu$g/mL).

From the equation, $K_d$ dissociation constant and maximum adsorption capability $\Gamma_{max}$ were calculated as 0.019 $\mu$g/mL and 286 $\mu$g/mg of PEI-PNIPAm microgel, respectively. The maximum amount of enzyme adsorbed at the standardized conditions on microgel was found to be 279 $\mu$g/mg. This value is very close to the maximum binding capacity of the PEI-PNIPAm micro gel which indicated that a high load of enzyme was achieved.

The respective concentration of enzyme at which maximum enzyme load was obtained was subsequently used for the preparation of bioconjugate and to carry out further studies. The linear graph (inset Figure 6(d)) of enzyme adsorption shows an excellent fit with relatively high $R^2$ values (0.99) and thus indicates that the model predicts the adsorption behavior very well.

Since the isoelectric point of the Acetyl CoA-synthetase used is around pH 7.5, at pH 8 the enzyme carries an overall negative charge, while the microgel carries a positive charge due to PEI on the surface of the microgel. These countercharged particles interact with each other through electrostatic forces and aid in the adsorption process leading to strong interactions between the enzyme and the PEI-PNIPAm microgel in a swollen state.

Example 6

Reaction kinetics of free and immobilized Acetyl CoA-synthetase

The reaction kinetics of free and immobilized enzymes was studied at various concentrations of acetate (5 mM - 0.3 mM) as a substrate and keeping concentrations of ATP and CoA constant at 1 mM and 0.5 mM, respectively. The activity of enzymes was studied in triplicate, and the standard deviation was used as the error.

The Michaelis-Menten equation provides important information regarding the rate of reaction and the Michaelis constant $K_m$ which is a direct measure of the enzyme-substrate affinity. These constants allow an assessment of the alterations in enzyme activity after immobilization such as a blocking of the active site, conformational changes or diffusion limitation.

Assuming Michaelis-Menten kinetics for Acetyl CoA-synthetase by the following equations:

$$V = \frac{V_{\max}[S]}{K_m + [S]} \qquad (8)$$

where V is the rate of the reaction, [S] is the concentration of the substrate, $K_m$ is the apparent constant, and $V_{max}$ is the maximum of reaction velocity. The values for $K_m$ and $V_{max}$, were estimated with the help of a Lineweaver-Burk plot for the Michaelis-Menten equation expressed as follows:

$$\frac{1}{V} = \frac{K_m}{V_{\max}} - \frac{1}{[S]} + \frac{1}{V_{\max}} \qquad (9)$$

The data obtained were managed and processed using Excel (Microsoft) and the parameters obtained from Lineweaver-Burk plot are represented in Table 1 and 2.

The $K_m$ value obtained for the free enzyme was close to the one obtained in earlier reports (T. Satyanarayana and Harold P. Klein. Studies on Acetyl-Coenzyme A Synthetase of Yeast: Inhibition by Long-Chain Acyl-Coenzyme A Esters. Journal Of Bacteriology, Aug. 1973, p. 600-606). In the case of PNIPAm-AEMA immobilization (Table 1), apparent $K_m$ values of acetate for immobilized enzyme was close to free enzyme. The Michaelis constant ($K_m$) is a direct measure of the enzyme substrate affinity. The marginal increase of $K_m$ of the immobilized enzyme indicates that the enzyme-substrate binding continues to be efficient. The maximum velocity ($V_{max}$) is the highest rate of substrate conversion, when the enzyme is fully saturated with substrate. For PNIPAm-AEMA immobilization, a 34% reduction in $V_{max}$ was observed for immobilized enzyme as compared to free enzyme. As $V_{max}$ is dependent on the active enzyme concentration, the decrease in maximum velocity may be due to inactivation of enzyme particles or due to limited diffusion of substrate to the immobilized enzyme.

Table 1. Kinetic parameters of PNIPAm-AEMA-immobilized and free enzyme for acetate substrate at 37°C.

| Enzyme | $K_m$ ($\mu$M) | $V_{max}$ (U/min) |
|---|---|---|
| Free enzyme | 170 $\pm$ 30 | $8.5 \times 10^{-3} \pm 0.4 \times 10^{-3}$ |
| Immobilized enzyme (PNIPAm-AEMA) | 188 $\pm$ 20 | $5.6 \times 10^{-3} \pm 0.1 \times 10^{-3}$ |

In the case of PEI-PNIPAm immobilization (Table 2), the $K_m$ values for the immobilized enzyme were high compared to free enzyme. The increase in $K_m$ after immobilization was mainly due to a low concentration of substrate near the active site of enzyme, which was similar in other cases reported for enzyme adsorption on PNIPAm microgels. But the activity of enzyme was markedly improved after adsorption on PNIPAm-PEI microgel which is indicated by more than twice increase in the $V_{max}$ value compared to free enzyme. This show an increase in catalytic efficiency of the enzyme after immobilization.

Table 2. Kinetic parameters of PEI-PNIPAm-immobilized and free enzyme for acetate substrate at 37°C.

| Enzyme | $K_m$ (mM) | $V_{max}$ (U/min) |
|---|---|---|
| Free enzyme | 180 $\pm$ 20 | $16 \times 10^{-3} \pm 1 \times 10^{-3}$ |
| Immobilized enzyme (PEI-PNIPAm) | 420 $\pm$ 10 | $44 \times 10^{-3} \pm 2 \times 10^{-3}$ |

Example 7

Effect of pH and temperature on free and immobilized Acetyl CoA-synthetase

Temperature and pH are two important parameters that influence activity of enzymes. It is important to study these factors to determine any change in conformation of an enzyme on binding to the support. Enzyme activities were assayed as described in Example 5 for assessing PNIPAm-AEMA immobilized enzyme and as described in Example 6 for assessing PEI-PNIPAm immobilized enzyme. Optimum pH condition for both immobilized and free enzymes was evaluated in the pH range 5 to 9 (PNIPAm-AEMA) or 6 to 9 (PEI-PNIPAm) and the properties of immobilized enzyme were compared with those of free enzyme.

The enzymes were assayed in 0.1 M 2-(N-morpholino)ethanesulfonic acid (MES) buffer (pH 5-6.5) and 0.1 M borate buffer (pH 7-9) at 37°C for 15 min at the respective pH. The effect of temperature on enzymes reactivity was studied at different temperatures ranging from 25°C to 55°C for PNIPAm-AEMA-immobilized enzyme and 25°C to 65°C for PEI-PNIPAm-immobilized enzyme in 0.1 M borate buffer (pH 8) for 15min. The enzymes' activity was normalized with respect to their maximum activity for given experimental condition and the final data were represented as relative activity.

It can be seen in Figure 7 (a) that the free enzyme has its optimal pH at 8.0 while for the PNIPAm-AEMA-immobilized enzyme the pH was shifted towards more alkalinity. For the study of PEI-PNIPAm-immobilized enzyme a similar situation can be observed in Figure 7(c). However, in this case the immobilized enzyme has a higher relative activity at pH 6 to 7 as well as over pH 8.5.

The electrostatic interactions of the enzyme with the matrix leads to unequal partitioning of $H^+$ and $OH^-$ between the microenvironment of the immobilized enzyme and the bulk phase.

This is attributed to the Donnan partition effect where charged support often leads to displacements in the pH activity profile of immobilized enzymes. Thus, the immobilized enzyme shows higher relative activity at pH values where the activity of the free enzyme declines significantly.

The temperature dependence of the activity of the enzyme was studied in the temperature range of 25°C to 55°C for PNIPAm-AEMA-immobilized enzyme and 25°C to 65°C for PEI-PNIPAm-immobilized enzyme. The results presented in Figure 7 (b) show that the immobilized enzyme was active at a broad temperature range and exhibited a temperature optimum of reaction at 37°C, similar to free enzyme. However, the immobilized enzyme is much more tolerant of higher temperatures up to 55°C. These results can be attributed to a rigidification of the protein conformation due to covalent immobilization of the enzyme making it less susceptible to the temperature-induced conformational changes. Additionally, the microenvironment of microgel-protein is another factor that increases the activity of the enzyme. This is because at an increased temperature the microgel structure collapses and provides improved substrate diffusion to the enzyme above the transition temperature of PNIPAm.

Figure 7 (d) shows a similar dependence of the activity of both the PEI-PNIPAm-immobilized and free enzyme on the temperature. The results also show that immobilized enzymes show a higher activity at lower temperatures and the activity gradually decreases with the increase in temperature. As the temperature reaches close to the LCST of the PNIPAm polymer there is pronounced collapse of the microgel core structure which may, cause sudden burst of enzyme and also breaking of ionic bonds between enzyme and PEI polymer. The loss in enzyme concentration results in the activity trend similar to free enzyme.

Example 8

Stability and reusability of PNIPAm-AEMA-immobilized Acetyl CoA-synthetase

The attachment of an enzyme to a suitable polymer matrix provides longer shelf life and thermal stability to the biocatalyst. Thermal stability of the enzyme after immobilization on a PNIPAm-AEMA microgel was checked at temperatures 32-60°C. The thermal stability of the conjugate enzyme was found to be comparatively higher than the free enzyme (Figure 8 (a)).

The enhancement of rigidity of enzyme structure upon attachment to a support resists change in the native conformation of enzyme. Protection of enzyme conformation from environmental changes due to restricted mobility of the covalently immobilized enzyme on support imparts enhanced thermal stability (see above).

In solution, the native enzyme tends to acquire a thermodynamically stable conformation which leads to distortion of structure and inactivation of enzyme. At a temperature below the LCST of polymer, PNIPAm-AEMA probably prevents the unfolding of the enzyme by forming a hydrated surface layer, like a protective colloid. Figure 8 (b) shows the data for storage stability of free and immobilized enzymes. The immobilized Acetyl CoA-synthetase maintains more than 90% of its initial activity after storage for 9 days at 4°C in 0.1 M borate buffer, while the free enzyme retains only 63% of its initial activity under similar conditions. This indicates that the storage stability of enzyme was improved remarkably after the immobilization on PNIPAm-AEMA core-shell microgel.

Reusability of a bioconjugate depends on the extent of stabilization which is directly related to the extent of covalent bond formation and electrostatic interactions. Immobilization of enzyme allows switching of a conjugate from soluble to insoluble form in solution enabling easy recovery from the reaction mixture. The operation stability of the PNIPAm-AEMA-immobilized ACS was studied to estimate the number of times an immobilized enzyme can be reused. To this end, consecutive operation cycles of enzymatic reaction were conducted for 15 min, conjugates were obtained as mentioned in immobilized enzyme preparation and further washed with 0.1 M borate buffer. The procedure was repeated using a fresh aliquot of substrate. From Figure 8 (c), it can be seen that the immobilized enzyme preserved up to 50% of its initial activity even after 4 consecutive operations.

Example 9

Stability and reusability of a PEI-PNIPAm-immobilized Acetyl CoA-synthetase membrane bioreactor

An enzymatic membrane reactor was constructed by covalently anchoring the immobilized enzyme-microgel conjugate on an already prepared microgel-PET support. The optimized bioconjugate of Acetyl CoA-synthetase on PEI-PNIPAm microgel was casted on a PET microgel membrane (see Figure 2).

The enzyme immobilized membrane surface was prepared on polyethylene terephthalate (PET) track etched membrane support using enzyme immobilized microgel. The membrane fabrication strategy is outlined in Figure 2. The pores of the PET membrane were first covered with $Cd(OH)_2$ nanostrands, which were prepared by mixing $CdCl_2$ and aminoethanol solution. An aqueous solution of 0.3 mM 2-aminoethanol was quickly mixed with an equivolume of 4 mM cadmium chloride and allowed to stand for 30 min to form cadmium hydroxide nanostrands.

The nanostrand solution (10 mL) was suction filtered on the PET membrane. Subsequently, a thin layer of microgel was prepared by filtering a 1 mg/ml concentration PEI-PNIPAm microgel solution and crosslinked by glutaraldehyde. After microgel crosslinking, the sacrificial cadmium hydroxide nanostrand layer was removed by repeated passing of 10 mM HCl solution.

Membrane was extensively rinsed with buffer and finally $100\mu l$ of (1.5 U) enzyme immobilized microgels were diluted in buffer and allowed to crosslink with free glutaraldehyde at 4°C for overnight on the prepared surface. The membrane was washed to remove the unbound bioconjugate and used in dead end filtration device to check the catalytic performance.

After successful membrane fabrication, a membrane bioreactor was prepared using a dead end filtration device and employed to study the biocatalytic synthesis of acetyl CoA.

The permeability behavior of the prepared membrane was assessed by recording the PBS buffer flux at varying trans-membrane pressure from 1 to 5 bar at room temperature. The flux versus pressure relationship showed linear correlation (Figure 9 (a)). Since the membrane showed sufficient flux at moderate transmembrane pressure, bioconversion efficiency was checked at 2 bar under constant stirring condition.

The prepared membrane was subjected to temperature studies and activity of immobilized enzyme with respect to time was monitored at two different operation temperatures, 25 and 37°C. The biocatalytic trend of the membrane presented in Figure 9 (b) indicates that initially the activity is almost similar but with the increment in time, the activity of the bioconjugate at 25°C was higher compared to at 37°C. This result was corresponding to the results obtained in temperature dependence studies of the bioconjugate PEI-PNIPAm-immobilized acetyl CoA-synthetase. Therefore further studies on membrane were carried out at 25°C.

The performance of the membrane was estimated by enzymatic bioconversion of acetate to acetyl CoA. 1.5U of enzymatic membrane was used for bioconversion of 5 mM of acetate and the reaction was carried out for a period of 24 hr with 5 mM of acetate. Figure 9 (c) represents the time dependent bioconversion of acetate, the conversion gradually increasing with time to obtain 70% conversion in 6 hr and finally 84% of total conversion was obtained at the end of 24 hr.

This indicates that the 1.5 U membrane reactor worked at $10\mu M$ per min conversion rate of acetate for an initial 6 hr period and after 24 hr the conversion rate remains still at $3\mu M$ per min. The prior art had reported a complete

conversion of 1 μM acetate in 1-2 min time using 6.12 U of reactor (Mannens, G., Siegers, G., Lambrecht, R., Claeys, A. Biochimica et Biophysics Acta 1988, 959, 214-219). The results obtained with the present invention are clearly superior to results published in the prior art.

Further the membrane was kept for storage stability at room temperature (23°C) and it was found that the membranes maintained >50% initial activity till the 4th day (data not shown). To study the efficacy of PEI-PNIPAm-immobilized enzyme as a reusable biocatalyst for acetyl CoA synthesis, the membrane was subjected for operation stability studies.

The catalyst reusability was determined by measuring stability of the enzyme on the membrane reactor as a function of the number of reuses. 8 consecutive cycles were operated with alternate washing steps. The residual activity after each cycle is depicted in Figure 9 (d).

The membrane showed consistent performance and maintained >70% initial activity of bioconjugate till the last cycle. Good operation stability of the membrane is due to the protective nature of PEI shell, which provides high stability to the biocatalyst. This stability allows multiple reuse of the reactor system of the present invention that is highly important for practical as well as commercial reasons.

**Claims**

1. Enzymatic reactor system comprising an enzyme capable of catalyzing the reaction

$$ATP + Acetate + CoA \rightarrow AMP + Pyrophosphate + Acetyl\text{-}CoA \qquad (1)$$

immobilized on a polymer microgel, wherein preferably said enzyme is an Acetyl-coenzyme A synthetase, wherein the polymer microgel comprises a cationic polymer.

2. Enzymatic reactor system according to claim 1, wherein the enzyme capable of catalyzing reaction (1) is an enzyme having the EC number 6.2.1.1.

3. Enzymatic reactor system according to claim 1 or 2, wherein the enzyme capable of catalyzing reaction (1) is a polypeptide having at least 80% identity, preferably at least 90% identity, more preferably at least 95%, even more preferably at least 99% identity, to a polypeptide having an amino acid sequence as set out in SEQ ID NO: 1 or in SEQ ID NO: 2.

4. Enzymatic reactor system according to any of claims 1 to 3, wherein the enzyme capable of catalyzing reaction (1) has the amino acid sequence as set out in SEQ ID NO: 1 (Acetyl-coenzyme A Synthetase 1; ACS1 from *Saccharomyces cerevisiae)* or in SEQ ID NO: 2 (Acetyl-coenzyme A Synthetase 2; ACS2).

5. Enzymatic reactor system according to any of claims 1 to 4, wherein the enzyme capable of catalyzing reaction (1) immobilized on a polymer microgel is supported on a membrane, preferably on a PET track etched membrane.

6. Enzymatic reactor system according to any of claims 1 to 5, wherein the polymer microgel comprises aminoethylmethacrylate and/or polyethyleneimine.

7. Enzymatic reactor system according to any of claims 1 to 6, wherein the polymer microgel comprises at least 0.001 wt%, preferably at least 0.05 wt%, more preferably at least 0.1 wt% of aminoethylmethacrylate and/or polyethyleneimine.

8. Enzymatic reactor system according to any of claims 1 to 7, wherein the polymer microgel comprises at most 5 wt%, preferably at most 1 wt%, more preferably at most 0.5 wt% of aminoethylmethacrylate and/or polyethyleneimine.

9. Enzymatic reactor system according to any of claims 1 to 8, wherein the polymer microgel further comprises poly(N-isopropylacrylamide).

10. Enzymatic reactor system according to any of claims 1 to 9, wherein the enzyme capable of catalyzing reaction (1) is immobilized on the polymer microgel by covalent linkage or ionic adsorption.

11. Enzymatic reactor system according to claim 10, wherein the enzyme capable of catalyzing reaction (1) is covalently linked to the microgel, preferably wherein the enzyme capable of catalyzing reaction (1) is covalently linked to functional groups on the surface of the microgel, more preferably the enzyme is covalently linked to amino groups on the surface of the microgel, even more preferably the enzyme is covalently linked to amino groups on the surface of the microgel comprising aminoethylmethacrylate.

12. Enzymatic reactor system according to claim 10, wherein the enzyme capable of catalyzing reaction (1) is immobilized on the microgel by ionic adsorption.

13. Enzymatic reactor system according to any of claims 1 to 12, wherein the polymer microgel is of the core-shell type.

14. Use of the enzymatic reactor system according to any of claims 1 to 13 for the synthesis of Acetyl-CoA, preferably wherein the system is used in a dead end filtration device.

15. Method for the preparation of an enzymatic reactor system according to any of claims 1 to 13, wherein the immobilization of the enzyme capable of catalyzing reaction (1) on a polymer microgel is performed by covalent linkage or ionic adsorption.

**Patentansprüche**

1. Enzymatisches Reaktorsystem, umfassend ein Enzym, welches zum Katalysieren der Reaktion

$$ATP + Acetat + CoA \rightarrow AMP + Pyrophosphat + Acetyl\text{-}CoA \qquad (1)$$

in der Lage ist, immobilisiert an einem Polymermikrogel, wobei das Enzym bevorzugt eine Acetyl-Coenzym A-Synthetase ist,
wobei das Polymermikrogel ein kationisches Polymer umfasst.

2. Enzymatisches Reaktorsystem gemäß Anspruch 1, wobei das Enzym, welches zum Katalysieren von Reaktion (1) in der Lage ist, ein Enzym mit der EC-Nummer 6.2.1.1 ist.

3. Enzymatisches Reaktorsystem gemäß Anspruch 1 oder 2, wobei das Enzym, welches zum Katalysieren von Reaktion (1) in der Lage ist, ein Polypeptid mit mindestens 80 % Identität, bevorzugt mindestens 90 % Identität, stärker bevorzugt mindestens 95 %, noch stärker bevorzugt mindestens 99 % Identität zu einem Polypeptid mit einer Aminosäuresequenz wie in SEQ ID Nr.: 1 oder in SEQ ID Nr.: 2 dargelegt ist.

4. Enzymatisches Reaktorsystem gemäß einem der Ansprüche 1 bis 3, wobei das Enzym, welches zum Katalysieren von Reaktion (1) in der Lage ist, die Aminosäuresequenz wie in SEQ ID Nr.: 1 (Acetyl-Coenzym A-Synthetase 1; ACS1 von *Saccharomyces cerevisiae*) oder in SEQ ID Nr.: 2 (Acetyl-Coenzym A-Synthetase 2; ACS2) dargelegt aufweist.

5. Enzymatisches Reaktorsystem gemäß einem der Ansprüche 1 bis 4, wobei das Enzym, welches zum Katalysieren von Reaktion (1) in der Lage ist, immobilisiert an einem Polymermikrogel, an einer Membran, bevorzugt an einer PET-Ätzspur-Membran getragen wird.

6. Enzymatisches Reaktorsystem gemäß einem der Ansprüche 1 bis 5, wobei das Polymermikrogel Aminoethylmethacrylat und/oder Polyethylenimin umfasst.

7. Enzymatisches Reaktorsystem gemäß einem der Ansprüche 1 bis 6, wobei das Polymermikrogel mindestens 0,001 Gew.-%, bevorzugt mindestens 0,05 Gew.-%, stärker bevorzugt mindestens 0,1 Gew.-% Aminoethylmethacrylat und/oder Polyethylenimin umfasst.

8. Enzymatisches Reaktorsystem gemäß einem der Ansprüche 1 bis 7, wobei das Polymermikrogel höchstens 5 Gew.-%, bevorzugt höchstens 1 Gew.-%, stärker bevorzugt höchstens 0,5 Gew.-% Aminoethylmethacrylat und/oder Polyethylenimin umfasst.

9. Enzymatisches Reaktorsystem gemäß einem der Ansprüche 1 bis 8, wobei das Polymermikrogel ferner Poly(N-isopropylacrylamid) umfasst.

10. Enzymatisches Reaktorsystem gemäß einem der Ansprüche 1 bis 9, wobei das Enzym, welches zum Katalysieren von Reaktion (1) in der Lage ist, an dem Polymermikrogel durch kovalente Bindung oder ionische Adsorption immobilisiert ist.

11. Enzymatisches Reaktorsystem gemäß Anspruch 10, wobei das Enzym, welches zum Katalysieren von Reaktion (1) in der Lage ist, kovalent an das Mikrogel gebunden ist, wobei das Enzyme, welches zum Katalysieren von Reaktion (1) in der Lage ist, bevorzugt kovalent an funktionelle Gruppen auf der Oberfläche des Mikrogels gebunden ist, wobei das Enzym stärker bevorzugt kovalent an Aminogruppen auf der Oberfläche des Mikrogels gebunden ist, wobei das Enzym noch stärker bevorzugt kovalent an Aminogruppen auf der Oberfläche des Mikrogels, umfassend Aminoethylmethacrylat, gebunden ist.

12. Enzymatisches Reaktorsystem gemäß Anspruch 10, wobei das Enzym, welches zum Katalysieren von Reaktion (1) in der Lage ist, an dem Mikrogel durch ionische Adsorption immobilisiert ist.

13. Enzymatisches Reaktorsystem gemäß einem der Ansprüche 1 bis 12, wobei das Polymermikrogel vom Kern-Schale-Typ ist.

**14.** Verwendung des enzymatischen Reaktorsystems gemäß einem der Ansprüche 1 bis 13 zur Synthese von Acetyl-CoA, wobei das System bevorzugt in einer Dead-End-Filtrationsvorrichtung verwendet wird.

**15.** Verfahren zur Herstellung eines enzymatischen Reaktorsystems gemäß einem der Ansprüche 1 bis 13, wobei die Immobilisierung des Enzyms, welches zum Katalysieren von Reaktion (1) in der Lage ist, an einem Polymermikrogel durch kovalente Bindung oder ionische Adsorption durchgeführt wird.

**Revendications**

**1.** Système de réacteur enzymatique comprenant une enzyme capable de catalyser la réaction

$$ATP + acétate + CoA \rightarrow AMP + pyrophosphate + acétyl\text{-}CoA \qquad (1)$$

immobilisée sur un microgel polymère, où ladite enzyme est de préférence, une acétyl-coenzyme A synthétase, où le microgel polymère comprend un polymère cationique.

**2.** Système de réacteur enzymatique selon la revendication 1, où l'enzyme capable de catalyser la réaction (1) est une enzyme numérotée EC 6.2.1.1.

**3.** Système de réacteur enzymatique selon la revendication 1 ou 2, où l'enzyme capable de catalyser la réaction (1) est un polypeptide ayant au moins 80 % d'identité, de préférence au moins 90% d'identité, de manière plus préférée au moins 95%, de manière encore plus préférée au moins 99% d'identité, avec un polypeptide ayant la séquence des acides aminés représentée en SEQ ID N°1 ou SEQ ID N°2.

**4.** Système de réacteur enzymatique selon l'une quelconque des revendications 1 à 3, où l'enzyme capable de catalyser la réaction (1) présente la séquence des acides aminés donnée en SEQ ID N°1 (acétyl-coenzyme A synthétase 1 ; ACS1 de *Saccharomyces cerevisiae*) ou en SEQ ID N°2 (acétyl-coenzyme A synthétase 2 ; ACS2).

**5.** Système de réacteur enzymatique selon l'une quelconque des revendications 1 à 4, où l'enzyme capable de catalyser la réaction (1) immobilisée sur un microgel polymère est supportée sur une membrane, de préférence une membrane en PET 'track-etched'.

**6.** Système de réacteur enzymatique selon l'une quelconque des revendications 1 à 5, où le microgel polymère comprend du méthacrylate d'aminoéthyle et/ou de la polyéthylèneimine

**7.** Système de réacteur enzymatique selon l'une quelconque des revendications 1 à 6, où le microgel polymère comprend au moins 0,001% en poids, de préférence au moins 0,05% en poids, de manière plus préférée au moins 0,1% en poids de méthacrylate d'aminoéthyle et/ou de polyéthylèneimine.

**8.** Système de réacteur enzymatique selon l'une quelconque des revendications 1 à 7, où le microgel polymère comprend au plus 5% en poids, de préférence au plus 1% en poids, de manière plus préférée au plus 0,5% en poids de méthacrylate d'aminoéthyle et/ou de polyéthylèneimine.

**9.** Système de réacteur enzymatique selon l'une quelconque des revendications 1 à 8, où le microgel polymère comprend en outre, le poly(N-isopropylacrylamide).

**10.** Système de réacteur enzymatique selon l'une quelconque des revendications 1 à 9, où l'enzyme capable de catalyser la réaction (1) est immobilisée sur le microgel polymère par liaison covalente ou adsorption ionique.

**11.** Système de réacteur enzymatique selon la revendication 10, où l'enzyme capable de catalyser la réaction (1) est liée de manière covalente au microgel, de préférence où l'enzyme capable de catalyser la réaction (1) est liée de manière covalente aux groupes fonctionnels sur la surface du microgel, de manière plus préférée l'enzyme est liée de manière covalente aux groupes amino sur la surface du microgel, de manière encore plus préférée, l'enzyme est liée de manière covalente aux groupes amino sur la surface du microgel comprenant du méthacrylate d'aminoéthyle.

**12.** Système de réacteur enzymatique selon la revendication 10, où l'enzyme capable de catalyser la réaction (1) est immobilisée sur le microgel par adsorption ionique.

13. Système de réacteur enzymatique selon l'une quelconque des revendications 1 à 12, où le microgel polymère est de type noyau-enveloppe.

14. Utilisation du système de réacteur enzymatique selon l'une quelconque des revendications 1 à 13, pour la synthèse d'acétyl-CoA, de préférence où le système est utilisé dans un dispositif de filtration frontale.

15. Procédé de préparation d'un système de réacteur enzymatique selon l'une quelconque des revendications 1 à 13, où l'immobilisation de l'enzyme capable de catalyser la réaction (1) sur un microgel polymère est réalisée par liaison covalente ou adsorption ionique.

Figure 1

PET membrane

Enzyme immobilized
membrane

Figure 2

**Figure 3**

*N*-Isopropyl
acrylamide

*N-N'*-Methylene
bisacrylamide

Polyethyleneimine

70 °C/ 4 h/ Ar
tBuOOH

PNIPAm-PEI
core shell microgel

Figure 4

Figure 5

**Figure 6**

**Figure 7**

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4610962 A
- EP 0084975 A2
- US 4960696 A
- GB 2215335 A

### Non-patent literature cited in the description

- **KONDO A. ; FUKUDA H.** *Journal of fermentation and bioengineering,* 1997, vol. 84, 337-341
- **JING XU ; FANG ZENG ; SHUIZHU WU ; XINXING LIU ; CHAO HOU ; ZHEN TONG.** *Nanotechnology,* 2007, vol. 18, 265704
- **MANNENS, G. ; SIEGERS, G. ; LAMBRECHT, R. ; CLAEYS, A.** *Biochimica et Biophysics Acta,* 1988, vol. 959, 214-219
- **MANNENS G. ; SLEGERS G. ; LAMBRECHT R. ; GOETHALS P.** *Radiopharmaceuticals,* 1988, vol. 25 (7), 695-705
- **SOKIC-LAZIC, D. ; MINTEER, S. D.** *Biosensors and Bioelectronics,* 2008, vol. 24, 939-944
- **MAN FAI LEUNG et al.** New Route to Smart Core-Shell Polymeric Microgels: Synthesis and Properties. *MACROMOLECULAR RAPID COMMUNICATIONS,* 03 November 2004, vol. 25 (21), 1819-1823
- **NICOLE WELSCH et al.** Enhanced Activity of Enzymes Immobilized in Thermoresponsive Core-Shell Microgels. *THE JOURNAL OF PHYSICAL CHEMISTRY B,* 10 December 2009, vol. 113 (49), 16039-16045
- Time warps, string edits and macromolecules: the theory and practice of sequence comparison. **KRUSKAL, J. B.** An overview of sequence comparison. Addison Wesley, 1983, 1-44
- **QIUGEN ZHANG et al.** Ultrathin freestanding nanoporous membranes prepared from polystyrene nanoparticles. *J. Mater. Chern.,* 2011, vol. 21, 1684
- **T. SATYANARAYANA ; HAROLD P. KLEIN.** Studies on Acetyl-Coenzyme A Synthetase of Yeast: Inhibition by Long-Chain Acyl-Coenzyme A Esters. *Journal Of Bacteriology,* August 1973, 600-606

SEQUENCE LISTING

<110> Leibniz-Institut fur Polymerforschung Dresden e.V.

<120> Enzymatic reactor system

<130> 3173-X-29483 EP

<160> 2

<170> BiSSAP 1.2

<210> 1
<211> 713
<212> PRT
<213> Saccharomyces cerevisiae

<400> 1

```
Met Ser Pro Ser Ala Val Gln Ser Ser Lys Leu Glu Glu Gln Ser Ser
1            5                   10                  15
Glu Ile Asp Lys Leu Lys Ala Lys Met Ser Gln Ser Ala Ala Thr Ala
            20                  25                  30
Gln Gln Lys Lys Glu His Glu Tyr Glu His Leu Thr Ser Val Lys Ile
            35                  40                  45
Val Pro Gln Arg Pro Ile Ser Asp Arg Leu Gln Pro Ala Ile Ala Thr
    50                  55                  60
His Tyr Ser Pro His Leu Asp Gly Leu Gln Asp Tyr Gln Arg Leu His
65                  70                  75                  80
Lys Glu Ser Ile Glu Asp Pro Ala Lys Phe Phe Gly Ser Lys Ala Thr
                85                  90                  95
Gln Phe Leu Asn Trp Ser Lys Pro Phe Asp Lys Val Phe Ile Pro Asp
            100                 105                 110
Pro Lys Thr Gly Arg Pro Ser Phe Gln Asn Asn Ala Trp Phe Leu Asn
            115                 120                 125
Gly Gln Leu Asn Ala Cys Tyr Asn Cys Val Asp Arg His Ala Leu Lys
    130                 135                 140
Thr Pro Asn Lys Lys Ala Ile Ile Phe Glu Gly Asp Glu Pro Gly Gln
145                 150                 155                 160
Gly Tyr Ser Ile Thr Tyr Lys Glu Leu Leu Glu Glu Val Cys Gln Val
                165                 170                 175
Ala Gln Val Leu Thr Tyr Ser Met Gly Val Arg Lys Gly Asp Thr Val
            180                 185                 190
Ala Val Tyr Met Pro Met Val Pro Glu Ala Ile Ile Thr Leu Leu Ala
    195                 200                 205
Ile Ser Arg Ile Gly Ala Ile His Ser Val Val Phe Ala Gly Phe Ser
    210                 215                 220
Ser Asn Ser Leu Arg Asp Arg Ile Asn Asp Gly Asp Ser Lys Val Val
225                 230                 235                 240
Ile Thr Thr Asp Glu Ser Asn Arg Gly Gly Lys Val Ile Glu Thr Lys
            245                 250                 255
Arg Ile Val Asp Asp Ala Leu Arg Glu Thr Pro Gly Val Arg His Val
            260                 265                 270
Leu Val Tyr Arg Lys Thr Asn Asn Pro Ser Val Ala Phe His Ala Pro
    275                 280                 285
Arg Asp Leu Asp Trp Ala Thr Glu Lys Lys Lys Tyr Lys Thr Tyr Tyr
    290                 295                 300
Pro Cys Thr Pro Val Asp Ser Glu Asp Pro Leu Phe Leu Leu Tyr Thr
305                 310                 315                 320
Ser Gly Ser Thr Gly Ala Pro Lys Gly Val Gln His Ser Thr Ala Gly
                325                 330                 335
Tyr Leu Leu Gly Ala Leu Leu Thr Met Arg Tyr Thr Phe Asp Thr His
            340                 345                 350
Gln Glu Asp Val Phe Phe Thr Ala Gly Asp Ile Gly Trp Ile Thr Gly
            355                 360                 365
His Thr Tyr Val Val Tyr Gly Pro Leu Leu Tyr Gly Cys Ala Thr Leu
370                 375                 380
Val Phe Glu Gly Thr Pro Ala Tyr Pro Asn Tyr Ser Arg Tyr Trp Asp
385                 390                 395                 400
```

```
Ile Ile Asp Glu His Lys Val Thr Gln Phe Tyr Val Ala Pro Thr Ala
            405                     410                 415
Leu Arg Leu Leu Lys Arg Ala Gly Asp Ser Tyr Ile Glu Asn His Ser
            420                 425                 430
Leu Lys Ser Leu Arg Cys Leu Gly Ser Val Gly Glu Pro Ile Ala Ala
            435             440                 445
Glu Val Trp Glu Trp Tyr Ser Glu Lys Ile Gly Lys Asn Glu Ile Pro
    450             455                 460
Ile Val Asp Thr Tyr Trp Gln Thr Glu Ser Gly Ser His Leu Val Thr
465             470             475                     480
Pro Leu Ala Gly Gly Val Thr Pro Met Lys Pro Gly Ser Ala Ser Phe
            485             490                 495
Pro Phe Phe Gly Ile Asp Ala Val Val Leu Asp Pro Asn Thr Gly Glu
        500             505                 510
Glu Leu Asn Thr Ser His Ala Glu Gly Val Leu Ala Val Lys Ala Ala
            515             520                 525
Trp Pro Ser Phe Ala Arg Thr Ile Trp Lys Asn His Asp Arg Tyr Leu
    530             535                 540
Asp Thr Tyr Leu Asn Pro Tyr Pro Gly Tyr Tyr Phe Thr Gly Asp Gly
545             550                 555                     560
Ala Ala Lys Asp Lys Asp Gly Tyr Ile Trp Ile Leu Gly Arg Val Asp
            565                 570                 575
Asp Val Val Asn Val Ser Gly His Arg Leu Ser Thr Ala Glu Ile Glu
            580             585                 590
Ala Ala Ile Ile Glu Asp Pro Ile Val Ala Glu Cys Ala Val Val Gly
            595             600                 605
Phe Asn Asp Asp Leu Thr Gly Gln Ala Val Ala Ala Phe Val Val Leu
    610             615                 620
Lys Asn Lys Ser Ser Trp Ser Thr Ala Thr Asp Asp Glu Leu Gln Asp
625             630                 635                     640
Ile Lys Lys His Leu Val Phe Thr Val Arg Lys Asp Ile Gly Pro Phe
            645                 650                 655
Ala Ala Pro Lys Leu Ile Ile Leu Val Asp Asp Leu Pro Lys Thr Arg
            660             665                 670
Ser Gly Lys Ile Met Arg Arg Ile Leu Arg Lys Ile Leu Ala Gly Glu
            675                 680                 685
Ser Asp Gln Leu Gly Asp Val Ser Thr Leu Ser Asn Pro Gly Ile Val
            690             695                 700
Arg His Leu Ile Asp Ser Val Lys Leu
705             710
```

<210> 2
<211> 683
<212> PRT
<213> Saccharomyces cerevisiae

<400> 2

```
Met Thr Ile Lys Glu His Lys Val Val Tyr Glu Ala His Asn Val Lys
1               5                   10                  15
Ala Leu Lys Ala Pro Gln His Phe Tyr Asn Ser Gln Pro Gly Lys Gly
            20                  25                  30
Tyr Val Thr Asp Met Gln His Tyr Gln Glu Met Tyr Gln Gln Ser Ile
        35                  40                  45
Asn Glu Pro Glu Lys Phe Phe Asp Lys Met Ala Lys Glu Tyr Leu His
    50                  55                  60
Trp Asp Ala Pro Tyr Thr Lys Val Gln Ser Gly Ser Leu Asn Asn Gly
65                  70                  75                  80
Asp Val Ala Trp Phe Leu Asn Gly Lys Leu Asn Ala Ser Tyr Asn Cys
            85                  90                  95
Val Asp Arg His Ala Phe Ala Asn Pro Asp Lys Pro Ala Leu Ile Tyr
        100                 105                 110
Glu Ala Asp Asp Glu Ser Asp Asn Lys Ile Ile Thr Phe Gly Glu Leu
    115                 120                 125
Leu Arg Lys Val Ser Gln Ile Ala Gly Val Leu Lys Ser Trp Gly Val
    130                 135                 140
Lys Lys Gly Asp Thr Val Ala Ile Tyr Leu Pro Met Ile Pro Glu Ala
145                 150                 155                 160
Val Ile Ala Met Leu Ala Val Ala Arg Ile Gly Ala Ile His Ser Val
```

```
                                165                           170                           175
         Val Phe Ala Gly Phe Ser Ala Gly Ser Leu Lys Asp Arg Val Val Asp
                     180                           185                           190
         Ala Asn Ser Lys Val Val Ile Thr Cys Asp Glu Gly Lys Arg Gly Gly
                     195                           200                           205
         Lys Thr Ile Asn Thr Lys Lys Ile Val Asp Glu Gly Leu Asn Gly Val
         210                           215                           220
         Asp Leu Val Ser Arg Ile Leu Val Phe Gln Arg Thr Gly Thr Glu Gly
         225                           230                           235                           240
         Ile Pro Met Lys Ala Gly Arg Asp Tyr Trp Trp His Glu Glu Ala Ala
                     245                           250                           255
         Lys Gln Arg Thr Tyr Leu Pro Pro Val Ser Cys Asp Ala Glu Asp Pro
                     260                           265                           270
         Leu Phe Leu Leu Tyr Thr Ser Gly Ser Thr Gly Ser Pro Lys Gly Val
                     275                           280                           285
         Val His Thr Thr Gly Gly Tyr Leu Leu Gly Ala Ala Leu Thr Thr Arg
                     290                           295                           300
         Tyr Val Phe Asp Ile His Pro Glu Asp Val Leu Phe Thr Ala Gly Asp
         305                           310                           315                           320
         Val Gly Trp Ile Thr Gly His Thr Tyr Ala Leu Tyr Gly Pro Leu Thr
                     325                           330                           335
         Leu Gly Thr Ala Ser Ile Ile Phe Glu Ser Thr Pro Ala Tyr Pro Asp
                     340                           345                           350
         Tyr Gly Arg Tyr Trp Arg Ile Ile Gln Arg His Lys Ala Thr His Phe
                     355                           360                           365
         Tyr Val Ala Pro Thr Ala Leu Arg Leu Ile Lys Arg Val Gly Glu Ala
         370                           375                           380
         Glu Ile Ala Lys Tyr Asp Thr Ser Ser Leu Arg Val Leu Gly Ser Val
         385                           390                           395                           400
         Gly Glu Pro Ile Ser Pro Asp Leu Trp Glu Trp Tyr His Glu Lys Val
                     405                           410                           415
         Gly Asn Lys Asn Cys Val Ile Cys Asp Thr Met Trp Gln Thr Glu Ser
                     420                           425                           430
         Gly Ser His Leu Ile Ala Pro Leu Ala Gly Ala Val Pro Thr Lys Pro
                     435                           440                           445
         Gly Ser Ala Thr Val Pro Phe Phe Gly Ile Asn Ala Cys Ile Ile Asp
         450                           455                           460
         Pro Val Thr Gly Val Glu Leu Glu Gly Asn Asp Val Glu Gly Val Leu
         465                           470                           475                           480
         Ala Val Lys Ser Pro Trp Pro Ser Met Ala Arg Ser Val Trp Asn His
                     485                           490                           495
         His Asp Arg Tyr Met Asp Thr Tyr Leu Lys Pro Tyr Pro Gly His Tyr
                     500                           505                           510
         Phe Thr Gly Asp Gly Ala Gly Arg Asp His Asp Gly Tyr Tyr Trp Ile
                     515                           520                           525
         Arg Gly Arg Val Asp Asp Val Val Asn Val Ser Gly His Arg Leu Ser
         530                           535                           540
         Thr Ser Glu Ile Glu Ala Ser Ile Ser Asn His Glu Asn Val Ser Glu
         545                           550                           555                           560
         Ala Ala Val Val Gly Ile Pro Asp Glu Leu Thr Gly Gln Thr Val Val
                     565                           570                           575
         Ala Tyr Val Ser Leu Lys Asp Gly Tyr Leu Gln Asn Asn Ala Thr Glu
                     580                           585                           590
         Gly Asp Ala Glu His Ile Thr Pro Asp Asn Leu Arg Arg Glu Leu Ile
                     595                           600                           605
         Leu Gln Val Arg Gly Glu Ile Gly Pro Phe Ala Ser Pro Lys Thr Ile
                     610                           615                           620
         Ile Leu Val Arg Asp Leu Pro Arg Thr Arg Ser Gly Lys Ile Met Arg
         625                           630                           635                           640
         Arg Val Leu Arg Lys Val Ala Ser Asn Glu Ala Glu Gln Leu Gly Asp
                     645                           650                           655
         Leu Thr Thr Leu Ala Asn Pro Glu Val Val Pro Ala Ile Ile Ser Ala
                     660                           665                           670
         Val Glu Asn Gln Phe Phe Ser Gln Lys Lys Lys
                     675                           680
```

18